**Europäisches Patentamt**

**European Patent Office**

**Office européen des brevets**

(19)

(11) Veröffentlichungsnummer: **0 001 999**
**B1**

(12) **EUROPÄISCHE PATENTSCHRIFT**

(21) Anmeldenummer: **78101261.2**

(22) Anmeldetag: **28.10.78**

(51) Int. Cl.³: **C 07 D 307/60**

(54) Verfahren zur Gewinnung von Maleinsäureanhydrid aus Destillationsrückständen

(30) Priorität: **10.11.77 DE 2750284**

(43) Veröffentlichungstag der Anmeldung:
**30.05.79 Patentblatt 79/11**

(45) Bekanntmachung des Hinweises auf die Patenterteilung:
**10.12.80 Patentblatt 80/25**

(84) Benannte Vertragsstaaten:
**BE DE FR GB NL**

(56) Entgegenhaltungen:
**DE - A - 2 061 335**
**DE - A - 2 061 336**
**GB - A - 876 549**
**US - A - 2 734 854**
**US - A - 2 770 630**
**US - A - 3 135 669**

(73) Patentinhaber: **BAYER AG**
**Zentralbereich Patente, Marken und Lizenzen**
**D - 5090 Leverkusen 1, Bayerwerk (DE)**

(72) Erfinder: **Heller, Karl-Heinz, Dr.**
**Scheiblerstrasse 103**
**D - 4150 Krefeld (DE)**
**Lenz, Günther, Dr.**
**Buschstrasse 165**
**D - 4150 Krefeld (DE)**

Courier Press, Leamington Spa, England.

## Verfahren zur Gewinnung von Maleinsäureanhydrid aus Destillationsrückständen

Die Erfindung betrifft ein Verfahren zur Gewinnung von Maleinsäureanhydrid aus maleinsäureanhydridhaltigen Destillationsrückständen, wie sie bei der technischen Aufarbeitung von rohem Maleinsäureanhydrid zu reinem Maleinsäureanhydrid anfallen.

So ist z.B. aus der DT—OS 2 061 335 und der DT—PS 2 061 336 ein Verfahren zur Gewinnung von reinem Maleinsäureanhydrid aus rohem Maleinsäureanhydrid, das durch Oxidation von Benzol oder $C_4$-Kohlenwasserstoffen hergestellt wurde, bekannt. Dabei verbleibt nach Abtrennen des reinen Maleinsäureanhydrids ein nach Maleinsäureanhydrid enthaltender Rückstand, aus dem Maleinsäureanhydrid durch Destillation zurückgewonnen wird. Ferner ist z.B. aus der US—PS 2 509 873, der US—PS 2 770 630 und der US—PS 2 989 545 bekannt, das bei der Herstellung von Phthalsäureanhydrid durch katalytische Oxidation von Naphthalin oder o-Xylol als Nebenprodukt anfallende Maleinsäureanhydrid aus dem Phthalsäureanhydrid-Abgas-Waschwasser durch Dehydratisierung und Destillation zu gewinnen. Dabei fällt nach der Abtrennung von reinem Maleinsäureanhydrid ebenfalls ein noch Maleinsäureanhydrid enthaltender Rückstand an. Nach diesen Verfahren ist es jedoch nicht möglich, das in den Destillationsrückständen enthaltende Maleinsäureanhydrid annähernd vollständig zurückzugewinnen.

In dem nach Durchführung der Destillation gemäß dem Stand der Technik verbleibenden Rückstand sind erfahrungsgemäß noch etwa 30 bis 50% Maleinsäureanhydrid enthalten. Dieser noch erhebliche Maleinsäureanhydrid-Gehalt ist notwenig, um den Rückstand genügend fließfähig zu halten, damit er aus der Destillationsapparatur abgelassen bzw. ausgespeist werden kann. Wird der Rückstand zu weit ausdestilliert, so steigen die Viskosität und der Gehalt an kristalliner Fumarsäure so stark an, daß der Rückstand nicht mehr in flüssiger Form aus der Destillationseinrichtung entfernt und der Vernichtung auf besonders vorteilhafte Weise durch z.B. Verbrennen in flüssiger Form zugeführt werden kann.

Die Verbrennung des Rückstandes in flüssiger Form stellt sowohl aus Kostengründen als auch aus Gründen des Umweltschutzes die günstigste Art seiner Beseitigung dar, da andere denkbare Methoden, wie z.B. Auflösen in Wasser und Beseitigung des Abwassers in einer biologischen Kläranlage oder durch Verbrennen der wäßrigen Lösung oder aber Erstarren lassen des Rückstandes und Deponieren oder Verbrennen in fester Form nach Zerkleinern, mit z.T. erheblichem Aufwand an Kosten verbunden sind bzw. eine Umweltbelastung dabei nicht völlig vermieden werden kann. Bei dem gemäß dem Stand der Technik durchgeführten Verfahren wird demnach ein erheblicher Teil (2 bis 8%) der produzierten Maleinsäureanhydrid-Menge mit dem Destillationsrückstand vernichtet und geht somit als Ausbeute verloren.

Es wurde nun ein Verfahren zur Gewinnung von Maleinsäureanhydrid aus Maleinsäureanhydrid-haltigen Destillationsrückständen durch Destillation gefunden, das dadurch gekennzeichnet ist, daß man Maleinsäureanhydrid aus dem Maleinsäureanhydrid enthaltenden Destillationsrückstand, der noch 30 bis 90% Maleinsäureanhydrid enthält, bis auf einen Endgehalt von weniger als 15 Gew.-% Maleinsäureanhydrid in einer Destillationseinrichtung ausdestilliert, wobei man dem Destillationsrückstand vor oder nach dem Ausdestillieren eine solche Menge eines Phthalsäureanhydridhaltigen Rückstandes, der sich aus 30 bis 80% Phthalsäureanhydrid, 10 bis 40% Benzoesäure, 3 bis 8% Maleinsäureanhydrid und 10 bis 20% höhersiedenden, teilweise harzartigen Substanzen zusammensetzt, zusetzt, daß das Gewichtsverhältnis von Phthalsäureanhydridhaltigem Rückstand zu ausdestilliertem Maleinsäureanhydrid-Rückstand 0,3 bis 1,5:1 beträgt, und anschließend das erhaltene Rückstandsgemisch aus dem Sumpf der Destillationseinrichtung austrägt.

Im besonderen wird Maleinsäureanhydrid aus dem Maleinsäureanhydrid-enthaltenden Destillationsrückstand bis auf einen Endgehalt von weniger als 5 Gew.-% Maleinsäureanhydrid ausdestilliert.

Der erfindungsgemäß dem Maleinsäureanhydrid-Destillationsrückstand vor oder nach dem Ausdestillieren zuzusetzende Phthalsäureanhydrid-Rückstand ist das bei der Destillation von Roh-Phthalsäureanhydrid zu Rein-Phthalsäureanhydrid in einer üblicherweise aus Vorlauf und Hauptlaufkolonne bestehenden Destillieranlage anfallende Gemisch aus Vorlauf und aus dem aus dem Sumpf der Hauptlaufkolonne ausgespeisten Sumpfprodukt. Das erfindungsgemäß verwendete und als Phthalsäureanhydrid-Rückstand bezeichnete Gemisch setzt sich im wesentlichen wie zuvor beschrieben zusammen und hat einen Erstarrungspunkt von etwa 100 bis 130°C.

Der Phthalsäureanhydrid-Rückstand ist bei Temperaturen von oberhalb 130°C niedrigviskos und enthält praktisch keine festen bestandteile. Er ist mit dem Maleinsäureanhydrid-Destillationsrückstand in jedem Verhältnis mischbar.

Der Phthalsäureanhydrid-Rückstand ist ein an sich wertloses Abfallprodukt der Phthalsäureanhydrid-Herstellung und wirdüblicherweise in einer Anlage zur Verbrennung flüssiger Abfallstoffe verbrannt.

Durch die Verwendung des Phthalsäureanhydrid-Rückstandes als Verdünnungsmittel für den Maleinsäureanhydrid-Rückstand wird einerseits bei der Rückstandsdestillation ein Maleinsäureanhydrid erhalten, das leicht zu einem spezifikationsgerechten Endprodukt verarbeitet werden kann, andererseits wird die weitgehend Maleinsäureanhydrid-freie Rückstandsmischung so niedrigviskos und gut handhabbar, daß man sie problemlos aus der Destillationseinrichtung ablassen und einer Verbrennungsanlage für flüssige Abfallstoffe zuführen kann.

Die Ausführung des erfindungsgemäßen Verfahrens kann beispielsweise folgendermaßen erfolgen. Bei der kontinuierlich oder diskontinuierlich durchgeführten Destillation einer Roh-Maleinsäureanhydrid-Lösung, die neben ca. 60 bis 80% Maleinsäureanhydrid etwa 15 bis 40% des bei der Dehydratisierung der wäßrigen Maleinsäureanhydridlösung verwendeten Schleppmittels, z.B. o-Xylol, sowie kleinere Mengen harzartiger Substanzen und schwerlöslicher Fumarsäure enthält, bleiben nach dem Abdestillieren des Schleppmittels sowie der Hauptmenge des Maleinsäureanhydrids ca. 5 bis 15%, bezogen auf abdestilliertes Maleinsäureanhydrid, eines Rückstandes zurück, der neben den hochsiedenden, teilweise harzartigen Bestandteilen und kristalliner Fumarsäure noch etwa 30 bis 90% Maleinsäureanhydrid enthält. Dieser Rückstand wird durch eine beheizte Rohrleitung mittels einer geeigneten Pumpe oder durch Unterdruck in eine diskontinuierlich betriebene Rückstandsdestillationseinrichtung gefördert. Diese Destillationseinrichtung besteht aus einem beheizbaren Rührkessel mit aufgesetztem Dämpferohr und daran angeschlossen einem warmwassergekühlten Kondensator, einer Vorlage und einer Vakuumeinrichtung. In diesem Destillationskessel wird dem Maleinsäureanhydrid-Destillationsrückständ der Phthalsäureanhydrid-Rückstand, entweder direkt über eine beheizte Leitung aus dem Rückstandabehälter einer Phthalsäureanhydrid-Destillationsanlage oder mittels eines beheizbaren Transportbehälters in einer Menge zugesetzt, die sich zur Menge des Maleinsäureanhydrid-Rückstandes wie etwa 0,3 bis 1,2 :1 verhält. In den meisten Fällen ist ein Mengenverhältnis von 0,4 bis 0,7 :1 ausreichend. Nach dem Mischen der beiden Rückstande wird das in der Mischung enthaltene Maleinsäureanhydrid unter einem Druck im Bereich von etwa 200—2,7 mbar, vorzugsweise 94,5—13,5 mbar und bei einem Sumpftemperaturbereich von etwa 100—180°C, vorzugsweise 140 bis 170°C, so weit abdestilliert, daß bei 26,7 mbar und 170°C kein Maleinsäureanhydrid mehr übergeht. Die Sumpfanalyse zeigt dabei Restgehalte an Maleinsäureanhydrid von unter 5%. Der weitgehend Maleinsäureanhydrid-freie Rückstand, der bei Temperaturen im Bereich von etwa 130—180°C niedrigviskos ist, wird durch eine Bodenablaßöffnung des Destillierkessels in einen beheizbaren Transportbehälter abgelassen und einer Verbrennungsanlage für flüssige Abfallstoffe zugeführt.

Das abdestillierte Maleinsäureanhydrid wird in den Roh-Maleinsäureanhydrid-Zwischenlagerbehälter zurückgepumpt und bei der nächsten Charge zu Rein-Maleinsäureanhydrid destilliert.

Die Ausbeute des Maleinsäureanhydrid-Aufarbeitungsprozesses wird durch das beschriebene erfindungsgemäße Verfahren um etwa 5 bis 8% erhöht.

In einer weiteren Ausführungsform des erfindungsgemäßen Verfahrens wird der bei der kontinuierlichen oder diskontinuierlichen Destillation der Roh-Maleinsäureanhydrid-Lösung erhaltene Rückstand, der noch ca. 30 bis 90% Maleinsäureanhydrid enthält, in die bereits beschriebene Rückstandsdestillationseinrichtung befördert und ohne Zusatz unter den oben angegebenen Bedingungen ausdestilliert bis kein Maleinsäureanhydrid mehr übergeht. Der verbleibende Rückstand, der weniger als 5% Maleinsäureanhydrid enthält, ist infolge seines relativ hohen Gehaltes an fester Fumarsäure und harzartiger Substanzen sehr schlecht fließfähig. Diesem Rückstand wird wie oben beschrieben bei einer Temperatur von etwa 160°C eine solche Menge des Phthalsäureanhydrid-Rückstandes zugesetzt, daß das Gew.-Verhältnis von Phthalsäureanhydrid-Rückstand zu ausdestilliertem Maleinsäureanhydrid-Rückstand etwa 0,5 bis 1,5 :1 beträgt. Diese jetzt sehr gut fließfähige Mischung wird bei Temperaturen im Bereich von etwa 130 bis 180°C abgelassen und mittels eines beheizbaren Transportbehälters der Verbrennung zugeführt.

Das abdestillierte Maleinsäureanhydrid wird der Roh-Maleinsäureanhydrid-Stufe des Aufarbeitungsprozesses zugeführt.

Wirtschaftliche Vorteile des erfindungsgemäßen Verfahrens ergeben sich demnach einmal durch die Erhöhung der Ausbeute des Maleinsäureanhydrid-Verfahrens in Höhe der aus dem Destillationsrückstand zurückgewonnenen Maleinsäureanhydrid-Menge, zum anderen durch die Verringerung der zu verbrennenden Rückstandsmenge, ebenfalls in Höhe der zurückgewonnenen Maleinsäureanhydrid-Menge. Der Wert des zugesetzten Phthalsäureanhydrid-Rückstandes kann vernachlässigt werden, da es sich um einen Abfallstoff handelt, der in jedem Fall sowieso verbrannt oder auf andere Weise, gegebenenfalls unter Verursachung zusätzlicher Kosten, vernichtet werden muß.

Die folgenden Beispiele dienen der weiteren Erläuterung des erfindungsgemäßen Verfahrens. Maleinsäureanhydrid wird in den Beispielen kurz als MSA bezeichnet.

### Beispiel 1

a) Durch Oxidation eines Buten-haltigen $C_4$-Kohlenwasserstoffgemisches mit Luftsauerstoff an einem in einem Röhrenreaktor angeordneten Festbettkatalysator wird ein MSA-haltiges Reaktionsgas erzeugt. In einem Gas-

wäscher wird durch Absorption und Hydratisierung des in dem Gasstrom enthaltenden MSA eine ca. 40%ige wäßrige Maleinsäurelösung erhalten. Bei der Dehydratisierung dieser Maleinsäurelösung unter Verwendung von o-Xylol als Schleppmittel in einer Dehydratisierkolonne fällt als Roh-MSA-Lösung eine Mischung von 80% MSA, ca. 18% o-Xylol und, neben anderen teilweise harzartigen Nebenprodukten, geringe Mengen an Fumarsäure an.

Diese Roh-MSA-Lösung wird in einer chargenweise betriebenen Destillationseinrichtung, im wesentliche bestehend aus einem Rührkessel mit Bodenkolonne, Kondensator, Vorlagen und Vakuumstation fraktioniert, wobei neben Vor- und Zwischenläufen reines MSA als Hauptprodukt gewonnen wird. Als Destillationsrückständ bleibt in einer Menge von 12%, bezogen auf produziertes Rein-MSA, eine Mischung, die laut Analyse z.B. folgende Zusammensetzung aufweist:

52% MSA

30% Harze

18% Fumarsäure

Dieser Rückstand wird mit einer Temperatur von 155°C in einen beheizbaren Transportbehälter abgelassen.

b) 3 t des MSA-Rückstandes werden jetzt in die Rückstandsdestillationseinrichtung eingefüllt. Die Rückstandsdestillationseinrichtung besteht aus einem 10 m³ Rührkessel, der mit einer 16-bar-Dampfbeheizung, Rührer, einem einfachen Destillationaufsatz ohne Kolonne, einem Kondensator, Produktvorlagen und Vakuumstation ausgerüstet ist. Dem MSA-Rückstand werden 2,5 t PSA-Rückstand aus dem Rückstandsbehälter einer PSA-Destillationsanlage mittels eines beheizbaren Transportbehälters zugegeben. Der PSA-Rückstand hat folgende Zusammensetzung: 62% PSA, 23% leichter siedende Verunreinigungen, im wesentlichen Benzoesäure, sowie als Rest ca. 15% höhersiedende, teils harzartige Substanzen.

Unter einem Vakuum von 40 mbar werden unter Rühren bei einer Sumpftemperatur von 130—160°C 1,37 t MSA abdestilliert, das nur geringfügig mit Benzoesäure und PSA verunreinigt ist (ca. 4%). Der verbleibende Rückstand hat einen MSA-Restgehalt von 4,6%. Er wird ohne Schwierigkeiten durch eine Bodenablaßöffnung des Destillierkessels in einen beheizbaren Transportbehälter abgelassen und läßt sich in einer Verbrennungsanlage für flüssige Abfallstoffe problemlos verbrennen.

Das abdestillierte MSA wird aus der Destillationsvorlage in den Roh-MSA-Zwischenlagerbehälter gepumpt. Die erreichte Erhöhung der MSA-Ausbeute des Gesamtprozesses beträgt 5,5%.

Beispiel 2

Eine Menge von 5 t des entsprechend Beispiel 1a) bei der Herstellung von MSA aus einem C₄-Gemisch erhaltenen MSA-Destillationsrückstand mit einem MSA-Gehalt von 52% wird in der in Beispiel 1b) beschriebenen Rückstandsdestillationseinrichtung unter Vakuum von 26,7 mbar bei Sumpftemperaturen von 130—170°C soweit ausdestilliert bis kein MSA mehr überdestilliert. Es werden 2,5 t MSA, weitgehend frei von Verunreinigungen, erhalten.

Zu dem verbliebenen Destillationsrückstand, der einen Rest-MSA-Gehalt von ca. 4% hat, werden 2 t PSA-Rückstand der in Beispiel 1b) angegebenen Zusammensetzung mit einer Temperature von 160°C zugefügt. Nach kurzem Vermischen bei 160°C kann die Rückstandsmischung problemlos in einen beheizten Transportbehälter abgelassen und der Verbrennung zugeführt werden. Das abdestillierte MSA (6% der produzierten MSA-menge) wird in den Roh-MSA-Zwischenbehälter zurückgefördert.

Beispiel 3 (Vergleichsbeispiel)

Eine weitere Menge von 5 t des nach Beispiel 1a) erhaltenen MSA-Destillationsrückständes mit einem MSA-Gehalt von 52% wird in die in Beispiel 1b) beschriebene Rückstandsdestillationseinrichtung eingefüllt. Unter einem Vakuum von 26,7 mbar und bei Sumpftemperaturen von 130—170°C wird solange destilliert bis kein MSA mehr überdestilliert. Der im Kessel verbliebene Rückstand enthält noch ca. 5% MSA. Er ist schlecht fließfähig und stark an kristalliner Fumarsäure angereichert. Er läßt sich bei Temperaturen von 160 bis 170°C nur zu einem geringen Teil aus dem Rührkessel ablassen, da sich die Ablauföffnung zusetzt. Eine Zuführung des abgelassenen Teils zur Verbrennung mittels Transportbehälter und beheizten Leitungen ist wegen Verstopfungen und mangels, Fließfähigkeit nicht möglich.

Beispiel 4

Das in einer Phthalsäureanhydrid-Produktionsanlage durch katalytische Oxidation von o-Xylol mit Luftsauerstoff erzeugte Reaktionsgas wird nach der Desublimation des darin als Hauptprodukt enthaltenen Phthalsäureanhydrids in einem Gaswäscher gereinigt. Dabei wird durch Absorption in Wasser und Hydratisierung des in dem Gasstrom ehthaltenen, als Nebenprodukt der o-Xylol-Oxidation gebildeten MSA eine ca. 40%ige Maleinsäurelösung erhalten. Aus dieser Maleinsäurelösung wird in einer Dehydratisierkolonne unter Verwendung von o-Xylol als Schleppmittel eine Roh-MSA-Lösung gewonnen, die ca. 80% MSA, 18% o-Xylol und, neben anderen teilweise harzartigen Nebenprodukten, geringe Mengen an Fumarsäure und Phthalsäure enthält.

Diese Roh-MSA-Lösung wird, wie in Beispiel 1a) beschrieben, chargenweise zu Rein-MSA destilliert. Als Destillationsrückstand bleibt in einer Menge von 15%, bezogen auf produziertes Rein-MSA, eine Mischung zurück, die etwa folgende Zusammensetzung aufweist:

61% MSA

25% Harze

12% Fumarsäure

2% Phthalsäureanhydrid

Dieser Rückstand wird mit einer Temperatur von 160°C in einen beheizbaren Transportbehälter abgelassen.

Eine Menge von 5 t dieses Rückstandes wird in der in beispiel 1b) beschriebenen Rückstandsdestillationseinrichtung unter einem Vakuum von 33,8 mbar bei Sumpftemperaturen von 130 bis 180°C soweit ausdestilliert bis kein MSA mehr überdestilliert. Es werden 2,95 t MSA erhalten.

Zu dem verbliebenen Destillationsrückstand, der einen Rest-MSA-Gehalt von 4,8% hat, werden 1,5 t PSA-Rückstand der in Beispiel 1b) angegebenen Zusammensetzung mit einer Temperatur von 170°C zugefügt. Nach kurzem Vermischen bei 160°C kann die Rückstandmischung vollständig in einen beheizbaren Transportbehälter abgelassen und der Verbrennung zugeführt werden.

## Patentansprüche

1. Verfahren zur Gewinnung von Maleinsäureanhydrid aus Maleinsäureanhydridhaltigen Destillationsrückständen durch Destillation, dadurch gekennzeichnet, daß man Maleinsäureanhydrid aus dem Maleinsäureanhydrid enthaltenden Destillationsrückständ, der noch 30 bis 90% Maleinsäureanhydrid enthält, bis auf einen Endgehalt von weniger als 15 Gew.-% Maleinsäureanhydrid in einer Destillationseinrichtung ausdestilliert, wobei man dem Destillationsrückständ vor oder nach dem Ausdestillieren eine solche Menge eines Phthalsäureanhydrid-haltigen Rückstandes, der sich aus 30 bis 80% Phthalsäureanhydrid, 10 bis 40% Benzoesäure, 3 bis 8% Maleinsäureanhydrid und 10 bis 20% höhersiedenden, teilweise harzartigen Substanzen zusammensetzt, zusetzt, daß das Gewichtsverhältnis von Phthalsäureanhydrid-haltigem Rückstand zu ausdestilliertem Maleinsäureanhydrid-Rückstand 0,3 bis 1,5:1 beträgt, und anschließend das erhaltene Rückstandsgemisch aus dem Sumpf der Destillationseinrichtung austrägt.

2. Verfahren nach Anspruch 1, dadurch gekennzeichnet, daß man die Destillation im Bereich von 100 bis 180°C bei einem Druck von 200 bis 2,7 mbar durchführt.

3. Verfahren nach Anspruch 1, dadurch gekennzeichnet, daß man das Rückstandsgemisch aus dem Sumpf der Destillationseinrichtung bei einer Temperatur von 130 bis 180°C austrägt.

## Claims

1. Process for the recovery of maleic anhydride from distillation residues containing maleic anhydride, by means of distillation, characterised in that maleic anhydride is distilled out of the maleic-anhydride-containing distillation residue, which still contains 30 to 90% maleic anhydride, in a distillation unit, until the final content of maleic anhydride is less than 15% by weight, wherein, before or after the distilling-out step a phthalic-anhydride-containing residue composed of 30 to 80% of phthalic anhydride, 10 to 40% of benzoic acid, 3 to 8% of maleic anhydride and 10 to 20% of higher-boiling, in some cases resinous substances, is added to the distillation residue in such an amount that the weight ratio of phthalic-anhydride-containing residue to the maleic anhydride residue which has been subjected to the distilling-out step is 0.3 to 1.5:1 and then the resulting mixture of residues is discharged from the bottom of the distillation unit.

2. Process according to Claim 1, characterised in that the distillation is conducted in the range from 100 to 180°C under a pressure of 200 to 2.7 mbars.

3. Process according to Claim 1, characterised in that the residual mixture is discharged from the bottom of the distillation unit at a temperature of 130 to 180°C.

## Revendications

1. Procédé de récupération d'anhydride maléique par distillation à partir de résidus de distillation qui contiennent de l'anhydride maléique, caractérisé en ce que, dans un appareil de distillation, on sépare par distillation l'anhydride maléique à partir du résidu de distillation contenant de l'anhydride maléique, lequel renferme encore 30 à 90% d'anhydride maléique, jusqu'à une teneur finale inférieure à 15% en poids d'anhydride maléique, en ajoutant au résidu de distillation, avant ou après la séparation distillatoire, une telle quantité d'un résidu contenant de l'anhydride phtalique se composant de 30 à 80% d'anhydride phtalique, 10 à 40% d'acide benzoïque, 3 à 8% d'anhydride maléique et 10 à 20% de substances à point d'ébullition élevé en partie résineuses, que le rapport pondéral du résidu contenant de l'anhydride phtalique au résidu d'anhydride maléique traité par distillation soit de 0.3 à 1,5:1, puis en ce qu'on décharge le

9

**0 001 999**

10

mélange de résidus obtenu à partir du fond de l'appareil de distillation.

2. Procédé selon la revendication 1, caractérisé en ce qu'on exécute la distillation dans l'intervalle de 100 à 180°C sous une pression de 200 à 2,7 mbars.

3. Procédé selon la revendication 1, caractérisé en ce qu'on décharge le mélange de résidus à partir du fond de l'appareil de distillation à une température de 130 à 180°C.